# EUROPEAN PATENT APPLICATION

(11) **EP 1 709 957 A2**
(43) Date of publication of application: **11.10.2006**
(21) Application number: 06006592.7
(22) Date of filing: 05.06.2002
(51) Int. Cl.: A61K 9/00, A61P 25/04, A61K 31/485, A61K 31/19, A61P 29/00

(54) **Sustained-release analgesic compounds**

(30) Priority: 05.06.2001 US 295556 P
(62) Divisional of application: 02734669.1
(71) Applicant: Control Delivery Systems, Watertown, MA 02472 (US)
(72) Inventor: Ashton, Paul, Boston, MA 02118 (US); Smith, Thomas, J., Altadena, CA 91101 (US); Cynkowski, Tadeusz, Brookline, MA 02445 (US); Cynkowska, Grazyna, Brookline, MA 02445 (US); Mickunas, Edmund, Holliston, MA 01746 (US)
(74) Representative: Vossius & Partner

(57) **Abstract**

A pharmaceutically active inventive compound comprises two independently active analgesic moieties covalently conjoined through a physiologically labile linker. A preferred embodiment comprises an opioid, such as morphine, covalently linked to at least one analgesic compound selected from the group consisting of an opioid or a no-opioid compound through a physiologically labile linker. Suitable covalent linkers are covalently bonded to the two independently active analgesic compounds through one or more lactone, lactam, or sulfonamido linkages. Suitable linkers include endogenous carboxylate, amido, and sulfonamido moieties, and exogenous moieties that form the aforementioned lactone, lactam or sulfonamido linkages.

## Description

### FIELD OF THE INVENTION

The present invention relates generally to compounds, compositions, articles of manufacture and methods for treating acute or chronic pain in a mammal. In particular, the present invention relates to a sustained release system that relieves local pain, while reducing or eliminating adverse systemic side effects.

### BACKGROUND OF THE INVENTION

The pain response is a protective reflex system, warning an individual of hostile situations and tissue injury. Although the following discussion focuses on pain management in humans, the person of skill in the art should appreciate that general concepts of pain are applicable to mammals in general, and that the concepts of pain management are applicable to veterinary medicine as well as to human medicine.

Pain may be classified by etiology, duration and severity. Etiologically, pain may be classified as somatogenic (i.e. organic) or psychogenic (occurring without associated organic pathology sufficient to explain the severity and/or duration of the pain). Somatogenic pain may be further sub-classified as nociceptive (arising out of stimulation of somatic or visceral pain-sensitive nerve fibers) or neuropathic (resulting from dysfunction of the nervous system).

With regard to duration, pain is generally categorized as either acute or chronic. In general, acute pain lasts, or is reasonably expected to last, less than one month, whereas chronic pain lasts for more than one month following acute tissue injury, or persists or recurs for more than three months. Chronic pain also includes pain that is expected to, or actually does, worsen or persist over time (such as pain associated with certain types of cancer). Chronic pain may be accompanied by gradual onset of loss of sleep, loss of appetite, anorexia, diminished libido, reduced bowel motility, and eventually depression.

Acute pain is often associated with surgery and with trauma. The intensity of acute postoperative pain varies considerably, depending on the extent of the surgical procedure performed, on the individual's pain sensitivity, and on the type of anesthetic management employed during surgery. In general, major operations on the thorax and the upper abdominal region induce the most intensive postoperative pain. Extensive orthopedic operations also produce strong postoperative pain.

The modem trend in pain management is toward prophylaxis, as it is generally both easier and more effective to prevent pain than it is to treat pain after the fact. Also, hormonal stress responses that accompany pain are considered harmful to the patient, and tend to impair the healing process and overall recovery. Pain is generally controlled by the administration of analgesic agents. Analgesic agents include opiates, agonistic-antagonistic agents, and anti-inflammatory agents.

The most commonly used pain treatment during the immediate postoperative period is the repeated administration of opiate analgesics, such as opioids, whether orally or parenterally (e.g. intravenously, intramuscularly, or subcutaneously). The potency of all opioids is roughly comparable and opioids are considered generally effective against the most severe pain with appropriate dosing at intervals. Opioids, however, have a wide variety of adverse side effects, which minimize their effectiveness.

The side effects associated with the use of opioids include respiratory depression, reduced cough reflex, bronchial spasms, nausea, vomiting, release of histamine, peripheral vasodilation, orthostatic hypotension, vagal impact on the heart, contraction of smooth muscles (sphincters), reduced peristaltic motility in the gastrointestinal tract, urinary retention, stimulated release of adrenaline and anti-diuretic hormone, changes in the regulation of body temperature and sleep pattem, tolerance and addiction.

The negative effects on respiratory function are of special importance to the postoperative mammalian patient During the course of major surgery under general anesthesia, a mammalian patient is typically put to sleep with anesthetic agents, is paralyzed with muscle relaxants, is intubated and placed on mechanical ventilation, and is given analgesic agents. All of these treatments have direct and indirect effects on depressing respiratory drive with the net consequence that postoperatively the mammalian patient may have trouble breathing. As opiates may cause respiratory depression, reduce the cough reflex, and cause bronchial spasms, it is not advisable to administer opiates to mammalian patients for pain control immediately after surgery to avoid impairing respiratory function. Conversely, the mammalian patient is deprived of effective postoperative pain control because the administration of opiates is contraindicated due to the impact on respiratory function.

In addition to the opioids, other classes of analgesic agents that are commonly used include agonistic-antagonistic analgesic agents, non-steroidal anti-inflammatory drugs, and psychoactive drugs. Agonistic-antagonistic analgesic agents are effective against moderate to severe pain, but due to their antagonistic properties, their analgesic efficacy does not increase by increasing the dosage above a certain level. Furthermore, higher doses of agonistic-antagonistic analgesic agents are often associated with unpleasant sympathomimetic and psychomimetic side effects such as tachycardia, increase in blood pressure, and agitation. However, the risk of respiratory depression also decreases in line with the diminished analgesic activity of the higher doses. Side effects of non-steroidal anti-inflammatory agents include gastrointestinal irritation, bronchospastic effects in asthmatic mammalian patients, and tinnitus.

Some chemical classes of non-opioid analgesics include, acetaminophen, indoles, such as etodolac (etodolic acid: an anti-inflammatory analgesic), and indomethacin (an anti-inflammatory, antipyretic, analgesic), naphthylalkanones, such as nabumetone (an anti-inflammatory analgesic), oxicams, such as piroxicam (an anti-inflammatory drug), propionic acids, such as fenoprofen, flurbiprofen, ibuprofen, ketoprofen, naproxen, naproxen sodium, and oxaprozin, salicylates, such as aspirin, choline magnesium trisalicylate, and diflunisal, fenamates, such as meclofenamic acid and mefenamic acid, and pyrazoles, such as phenylbutazone. Newer cyclooxygenase II selective inhibitors (COX-2), such as rofecoxib, and celecoxib, are likewise anti-inflammatory analgesic drugs, with indications for long-term treatment of pain.

NSAIDs are particularly valuable in treatment and prevention of post-operative pain, as they effectively inhibit cyclooxygenase, thereby reducing prostaglandin synthesis that is an essential part of the inflammation response. Aspirin, the classic **NSAID,** has been joined by a large number of other NSAID drugs, such as ibuprofen, flurbiprofen, ketoprofen, naproxen, and fenoprofen. At least a substantial number of patients, however, are excessively sensitive to NSAIDs, with reactions ranging from mild tinnitus, to moderate to severe bowel disturbance, e.g. peptic ulceration, to extreme anaphylaxis. The clinical usefulness of NSAIDs is further restricted by other potential adverse effects, including hepatic necrosis, granulomatous hepatitis, cholestatic hepatitis, transient increases in serum aminotransferases; fluid retention, and decreased sodium excretion, followed by hyperkalemia, liguria and amiva. As moderate to severe bowel disturbance is by far the most common side effect responsible for patient avoidance of NSAID therapy, non-oral administration routes would appear to be particularly attractive for at least a sizeable minority of persons recovering from surgery. However, parenteral modes of administration, such as intravenous, intramuscular and subcutaneous injection have proven less than ideal, inasmuch as they require such inconveniences ranging from the mild (professional nursing care) to the extreme (a central line) for their administration.

As mentioned above, the anti-inflammatory property of NSAIDs makes them particularly attractive as therapeutic agents for treatment and prophylaxis of pain. Other anti-inflammatory drugs include corticosteroids, which have shown great promise in topical routes of administration. However, the systemic effects of corticosteroids greatly reduces their suitability for treatment of post-operative and deep-tissue injury related pain.

Other negative side effects associated with analgesic agents include tolerance and eventual physical dependency on these analgesic agents, especially the opioids, thereby reducing the effectiveness of the pain treatment and continuing the suffering from the chronic pain.

Examples of opioid drugs include codeine, fentanyl, hydromorphone, levorphanol, meperidine, morphine, oxycodone, oxymorphone, propoxyphene, buprenorphine, butorphanol, dezocine, nalbuphine, and pentazocine. These agents vary with respect to half-life, systemic versus peripheral activity, tendency to be metabolized to form undesirable metabolites, tendency to cause dysphoria, tendency to induce opioid tolerance and physical or psychological addiction.

For example, the withdrawal of morphine, heroin, or other opioid agonists with similar duration of action from an individual dependent upon the opioid gives rise to lacrimation, rhinorrhea, yawning, and sweating 8 to 12 hours after the last dose of the opioid. As withdrawal progresses, the individual will be subject to dilated pupils, anorexia, gooseflesh, restlessness, irritability, and tremor. At the peak intensity of withdrawal, which is 48 to 72 hours for morphine and heroin, the individual suffers from increasing irritability, insomnia, marked anorexia, violent yawning, severe sneezing, lacrimation, coryza, weakness, depression, increased blood pressure and heart rate, nausea, vomiting, intestinal spasm, and diarrhea. The individual commonly experiences chills alternating with hot flashes and sweating, as well as abdominal cramps, muscle spasms and kicking movements, and pains in the bones and muscles of the back and extremities, and exhibits leukocitosis and exaggerated respiratory response to carbon dioxide. Typically the individual does not eat or drink, which, when combined with the vomiting, sweating, and diarrhea, results in weight loss, dehydration, and ketosis. The withdrawal symptoms from morphine and heroin usually disappear in 7 to 10 days, but the drug dependent individual suffers greatly during the withdrawal period. If an opioid antagonistic drug is administered to the individual, such as naloxone, withdrawal symptoms develop within a few minutes after parenteral administration and reach peak intensity within 30 minutes, with a more severe withdrawal than from withholding the opioid. Withdrawal of morphine-like opioids will produce the same or similar withdrawal symptoms, with the intensity of the symptoms dependent upon the duration of action of the morphine-like opioid.

The drug withdrawal symptoms and the pain associated with them will be alleviated if a suitable opioid is given to the individual, however, this could result in the individual merely substituting dependency on one opioid for another. In the case of individuals dependent upon opioids such as morphine or heroin, methadone, an opioid with morphine-like activity, is given to the drug dependent individual on a daily basis. The methadone suppresses the opioid withdrawal symptoms and diminishes the euphoric effects of all opioids, but if the methadone is abruptly withdrawn, withdrawal symptoms similar to those from morphine will appear, albeit of less intensity but more prolonged.

All of the above-mentioned non-steroidal analgesic compounds (e.g. NSAIDs, opioid narcotics, etc.) possess the disadvantage of being relatively soluble in physiologic solutions, such as blood plasma, and are therefore ill-suited for controlled-release applications, whereby analgesic compounds are injected into the vicinity of a pain-causing trauma to prevent, control or reduce pain. Some steroidal anti-inflammatory drugs suffer from the same disadvantage as the non-steroidal analgesics, while others, such as some corticosteroids, are suitable for long-term relief when administered intramuscularly. However, the tendency for steroids to have systemic effects, such as immunosuppressive effects, makes them less-than-desirable for post-operative pain management.

Accordingly, there is a need for compounds and compositions useful for the prevention, treatment, amelioration or reduction of pain in an individual, that are relatively insoluble in physiologic fluids, such as blood plasma.

There is further a need for compounds and compositions that are stable before being exposed to physiologic fluids, such as blood plasma, that are relatively insoluble in physiologic fluids, such as blood plasma, and which, when dissolved into physiologic fluids, such as blood plasma, revert to one or more analgesic compounds or compositions.

There is further a need for compounds and compositions that are useful for sustained-release of analgesic agents in the vicinity of a wound, insult, injury, trauma, surgical incision, or other source of pain in a human or a non-human animal.

There is further a need for a method of treating an individual experiencing or expectirig to experience acute or chronic pain, wherein one or more analgesic agents are released in the vicinity of a pain locus in the individual, in concentrations effective to prevent, treat, ameliorate or reduce pain, while avoiding untoward systemic effects.

There is further a need for a method, a composition and a compound for the treatment of acute or chronic pain, which provides effective control of pain without the harmful side effects associated with traditional analgesics, such as respiratory depression, disturbed sleep patterns, decrease in appetite, and physical dependency, addiction to narcotic drugs, pain associated with withdrawal symptoms, and dependency on the replacement drug, methadone, for the narcotic drug.

### SUMMARY OF THE INVENTION

The foregoing and other needs are met by embodiments according to the present invention, which provide compounds comprising a first analgesic moiety covalently linked to at least a second analgesic moiety through a physiologically labile linker, or a salt thereof.

The foregoing and other needs are met by embodiments according to the present invention, which provide pharmaceutical compounds comprising a first analgesic moiety covalently linked to at least a second analgesic compound through a physiologically labile linker, or a pharmaceutically acceptable salt thereof.

The foregoing and other needs are met by embodiments according to the present invention, which provide methods of preventing or relieving pain in an individual in need of pain relief or prevention, comprising administering to the individual a pharmaceutical compound, comprising a first analgesic moiety covalently linked to at least a second analgesic moiety through a physiologically labile linker, or pharmaceutically acceptable salts thereof.

One aspect of the invention relates to a sustained release analgesic system comprising a prodrug having a general formula of A-L-B in which: A represents an non-steroidal anti-inflammatory drug (NSAID) moiety or an opioid drug moiety having a therapeutically active form for producing an analgesic response in a patient; L represents a covalent linker linking A and B to form a prodrug, said linker being cleaved under physiological conditions to generate said therapeutically active form of A; and B represents a moiety which, when linked to A, results in the prodrug having a lower solubility than the therapeutically active form of A alone.

Another aspect of the present invention provide a sustained release analgesic system comprising a prodrug having a general formula of A::B in which A represents an NSAID or opioid drug moiety having a therapeutically active form for producing an analgesic response in a patient; "::" represents an ionic bond between A and B that dissociates under physiological conditions to generate said therapeutically active form of A; and B represents a moiety which, when ionically bonded to A, results in the prodrug having a lower solubility than the therapeutically active form of A.

In certain embodiments, the linkage L is hydrolyzed in bodily fluid. In other embodiments, the linkage L is enzymatically cleaved. Examples of linkages which can be used include one or more hydrolyzable groups selected from the group consisting of an ester, an amide, a carbamate, a carbonate, a cyclic ketal, a thioester, a thioamide, a thiocarbamate, a thiocarbonate, a xanthate and a phosphate ester.

In preferred embodiments, the subject compositions are sterile and pyrogen free.

In certain preferred embodiments, the therapeutically active form of A is at least 5 times more soluble in water relative to said prodrug, and even more preferably at least 10, 50 or even 100 times more soluble in water relative to said prodrug.

In certain preferred embodiments, the therapeutically active form of A has a logP value at least 0.5 logP unit less than the logP value of the prodrug, and even more preferably at least 1, 1.5 or even 2 logP unit less than the logP value of the prodrug.

In certain preferred embodiments, the prodrug, in its linked form, has an ED50 for producing analgesia at least 10 times greater than the ED50 of the therapeutically active form of A, and even more preferably at least 100,1000 or even 10000 times greater than the ED50 of the therapeutically active form of A. That is, in many embodiments, the prodrug per se is inert with respect to inducing analgesia.

In certain embodiments, B is a hydrophobic aliphatic moiety. For instance, B, after cleavage from the prodrug, can be a biologically inert moiety. In other embodiments, B is another analgesic drug moiety having a therapeutically active form generated upon cleavage of said linker L or dissociates of said ionic bond, and may be the same drug or a different drug than A. Merely to illustrate, B can be an NSAID or an opiate.

In certain embodiments, B is an opioid. For instance, the opioid can be selected to have an active form selected from the group consisting of in the apomorphine, buprenorphine, codeine, dihydrocodeine, dihydroetorphine, diprenorphine, etorphine, hydrocodone, hydromorphone, levorphanol, meperidine, metopon, o methylnaltrexone, morphine, naloxone, naltrexone, normorphine, oxycodone, and oxymorphone. In other embodiments, the opioid is fentanyl or a fentanyl derivative. In other embodiments, the opioid is selected from the group consisting of affentanil, β-hydroxy-3-methylfentanyl, 4-methoxymethylfentanyl, 4-methyl fentanyl, carfentanil, fentanyl, lofentanil, meperidine, remifentanif, and sufentanil. In preferred embodiments, the active form of the opioid is an analgesic opioid.

In certain embodiments, A is an NSAID moiety represented in the general formula: 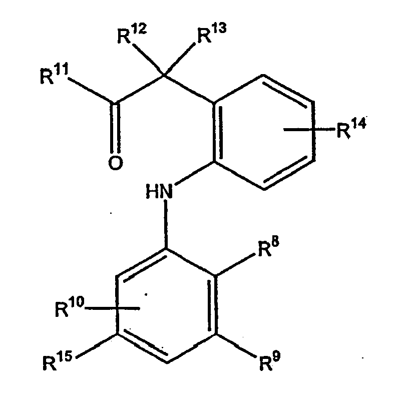 wherein
R8 is a lower alkyl, a lower alkoxy, a fluoro, a chloro;
R9 and R10 are each, independently for each occurrence, a hydrogen, a lower alkyl, a fluoro, a chloro, or a trifluoromethyl;
R11 is a hydrogen, a lower alkyl or benzyl;
R12 is a hydrogen or a lower alkyl;
R13 is a hydrogen, a lower alkyl or when R12 is hydrogen, benzyl;
R14 is a hydrogen, a lower alkyl, a lower alkoxy, a fluoro, a chloro, or a bromo;
R15 is hydrogen or trifluoromethyl when R8 is hydrogen or chloro and R9 is hydrogen or trifluoromethyl.

In certain embodiments, the NSAID is selected from piroxicam, diclofenac, etodolac, indomethacin, ketoralac, oxaprozin, tolmetin, naproxen, flubiprofen, fenoprofen, ketoprofen, ibuprofen, mefenamic acid, sulindac, apazone, phenylbutazone, aspirin, celecoxib and rofecoxib, and derivatives thereof. In certain preferred embodiments, the active form of the NSAID is a benzeneacetic acid derivative, such as diblofenac or a diclofenac derivative.

In other preferred embodiments, the NSAID is a a member of the arylacetic acid group of NSAIDs, such as naproxen or a naproxen derivative.

Another aspect of the invention provides compounds (co-drugs) of the formula:

A1-L-A2

wherein
L is a linking group, that covalently links A₁ and A₂ through at least one physiologically labile covalent bond;
A₁ is a residue of a first analgesic compound; and
A₂ is a residue of a second analgesic compound that may be the same as or different from A₁.

In certain preferred embodiments, the therapeutically active form of A1 and/or A2
is at least 5 times more soluble in water relative to said co-drug, and even more preferably at least 10, 50 or even 100 times more soluble in water relative to said co-drug.

In certain preferred embodiments, the therapeutically active form of A1 and/or A2
has a logP value at least 0.5 logP unit less than the logP value of the co-drug, and even
more preferably at least 1,1.5 or even 2 logP unit less than the logP value of the co-drug.

The foregoing and other needs are met by embodiments according to the present invention, which provide an article of manufacture comprising a compound comprising a first analgesic moiety covalently linked to at least a second analgesic moiety through a physiologically labile linker, and a polymer matrix.

The foregoing and other needs are met by embodiments according to the present invention, which provide an analgesic method of treating an individual in need of analgesic therapy, comprising administering to said individual an analgesically effective amount of a composition comprising a compound comprising a first analgesic moiety covalently linked to a second analgesic moiety via a physiologically labile linker.

The foregoing and other needs are met by embodiments according to the present invention, which provide compounds of the following formula: 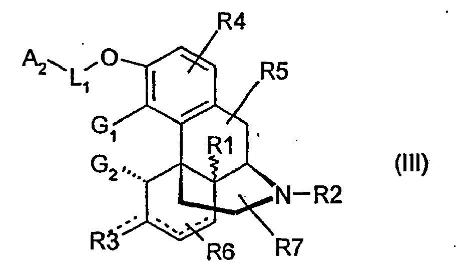 wherein A₂ is a second analgesic moiety;
L₁ is a direct bond or a linker;
Each ― is, as valence permits, a single or a double bond;
R₁ is H, CH₃ or OH;
R₂ is H, C₁-C₆-alkyl, C₃-C₆-cycloalkyl-C₁-C₆-alkyl, C₁-C₆-alkenyl, C₁-C₆₋alkanoyl, C₃-C₆-cycloalkenyl-C₁-C₆alkyl, C₃-C₆-cycloalkyl-C₁-C₆-alkanoyl, or C₃-C₆₋cycloalkenyl-C₁-C₆-alkanoyl;
R₃ is H, oxo. (=O), hydroxyl (-OH), or C₁-C₁₂-alkanoyl, or-L₂-A₃;
wherein L₂ is a direct bond or a linker and A₃ is a residue of an analgesic compound, which may be the same as, or different from, A₁ and A₂;
R₄-R₇ are H, methyl, ethyl, F, Cl, Br, or l;
and G₁ and G₂ are each H or together represent the oxygen of a dihydrofurano ring or salts thereof.

These and other aspects, properties, objects and advantages of the present invention will become clear to the person having ordinary skill in the art upon consideration of the following description, examples, and claims.

### DETAILED DESCRIPTION OF THE INVENTION

The present invention provides compounds for treatment and/or prevention of pain, pharmaceutical compositions comprising those compounds, articles of manufacture comprising the compounds in combination with a polymer matrix, and methods of treating pain comprising administering the compounds to individuals in need of analgesic treatment.

The present invention provides a class of analgesic compounds, each of which comprises a first analgesic and a second analgesic moiety, the two analgesic moieties being linked to one another via a physiologically labile linkage. The two analgesic moieties may be the same or different, and in fact may be from entirely different classes of analgesics. For instance the first analgesic moiety may be an opioid, while the second analgesic moiety may be a non-steroidat anti-inflammatory (NSAID).

The physiologically labile linkage may be any linkage that is labile under conditions approximating those found in physiologic fluids, such as blood plasma. The linkage may be a direct bond (for instance, an amide, ester, carbonate, carbamate, sufonate, or a sulfamate linkage) or may be a linking group (for instance a C₁-C₁₂ dialcohol, a C₁-C₁₂ hydroxylalkanoic acid, a C₁-C₁₂ hydroxyalkylamine, a C₁₋C₁₂ diacid, a C₁-C₁₂ aminoacid, or a C₁-C₁₂ diamine). Especially preferred linkages are direct amide, ester, carbonate, carbamate, and sulfamate linkages, and linkages via succinic acid, salicylic acid, diglycolic acid, oxa acids, oxamethylene, and halides thereof. The linkages are labile under physiologic conditions, which generally means pH of about 6 to about 8. The lability of the linkages depends upon the particular type of linkage, the precise pH and ionic strength of the physiologic fluid, and the presence or absence of enzymes that tend to catalyze hydrolysis reactions in vivo. In general, lability of the linkage in vivo is measured relative to the stability of the linkage when the compound has not been solubilized in a physiologic fluid. Thus, while some compounds according to the present invention may be relatively stable in some physiologic fluids, nonetheless, they are relatively vulnerable to hydrolysis in vivo (or in vitro, when dissolved in physiologic fluids, whether naturally occurring or simulated) as compared to when they are neat or dissolved in non-physiologic fluids (e.g. non-aqueous solvents such as acetone). Thus, the labile linkages are such that, when the drug is dissolved in an aqueous solution, especially a physiologic fluid such as blood plasma, the reaction is driven to the hydrolysis products.

The first and second analgesic moieties may be the same or different, and are generally speaking any analgesic moieties that either possess, or may be adapted to possess, a group that may be condensed with a linkage to form a hydrolytically labile bond. Examples of such groups are hydroxy (-OH, or alcohol) groups, amine (-NH₂) groups, acid (-COOH) groups, sulfonamide (-SO₂NH₂) groups, and sulfonate (-SO₃H) groups. The first and second analgesic moieties may be selected from opioids, non-steroidal anti-inflammatory agents, steroidal anti-inflammatory agents, and other analgesics, such as para-aminophenol derivatives like acetaminophen.

In some embodiments according to the present invention, the first analgesic moiety is selected from the group consisting of opiates such as codeine, fentanyl, hydromorphone, levorphanol, meperidine, morphine, oxycodone, oxymorphone, propoxyphene, buprenorphine, butorphanol, dezocine, nalbuphine, pentazocine, NSAIDs such as diclofenac, etodolac, indomethacin, sulindac, tolmetin, nabumetone, piroxicam, acetaminophen, fenoprofen, flurbiprofen, ibuprofen, ketoprofen, naproxen, oxaprozin, aspirin, choline magnesium trisalicylate, diflunisal, meclofenamic acid, mefenamic acid, phenylbutazone, glucocorticosteroids such as fluocinolone acetonide, prednisolone, prednisolohe tertiary-butylacetate, triamcinolone acetonide, hydrocortisone, dexamethasone, or prodrugs or active metabolites thereof. In a preferred embodiment according to the present invention, the first analgesic agent is morphine.

In some embodiments according to the present invention, the second analgesic agent is selected from the group consisting of opiates such as codeine, fentanyl, hydromorphone, levorphanol, meperidine, morphine, oxycodone, oxymorphone, propoxyphene, buprenorphine, butorphanol, dezocine, nalbuphine, pentazocine, NSAIDs such as diclofenac, etodolac, indomethacin, sulindac, tolmetin, nabumetone, piroxicam, acetaminophen, fenoprofen, flurbiprofen, ibuprofen, ketoprofen, naproxen, oxaprozin, aspirin, choline magnesium trisalicylate, diflunisal, meclofenamic acid, mefenamic acid, phenylbutazone, fluocinolone acetonide, prednisolone, prednisolone tertiary-butylacetate, dexamethasone, or prodrugs or active metabolites thereof. In preferred embodiments according to the present invention, the second analgesic agent is selected from morphine, naproxen, fluocinolone acetonide, aspirin, flurbiprofen, indomethacin, and naproxen, and diclofenac.

As mentioned above, the first and second analgesic moieties may be covalently linked either by a direct linkage or by an indirect linkage, through a linker. This relationship is generically expressed in the following formula (I):

A₁-L-A₂ (I)

wherein A₁ and A₂ are the residues of the first and second analgesic moieties, respectively, as defined above, and the L group is either a direct bond or a linker as described above. When L is a direct bond, the formula (I) above may be expressed more compactly as formula (II):

A₁-A₂ (II)

wherein A₁ and A₂ are as defined above.

When a compound of formula I is exposed to physiologic fluids, such as blood plasma, it is subjected to hydrolysis according to equation (1).

A₁-L-A₂ + 2H₂O = A₁ + A₂ + L (1).

In the special case where L is a direct bond, there is no L on the right side of the equation, and equation (1) collapses to equation (2):

A₁-A₂ = A₁ + A₂ (2).

The person having ordinary skill in the art will recognize that in formulae (I) and (II), and on the left-hand sides of equations (1) and (2), A₁ and A₂ stand for the residues of the first and second analgesic moieties (i.e. that portion of the analgesic drug according to the present invention corresponding to the first and second analgesic moieties themselves as released upon hydrolysis), whereas on the righthand sides of equations (1) and (2), A₁ and A₂ stand for the first and second analgesic moieties, as defined above, themselves. This convention has been adopted in the appended claims, and the person having skill in the art should understand in the context of the present invention, that when A₁ and A₂ stand alone, they stand for the first and second analgesic moieties, respectively, and when they are linked in a formula, such as formula (I) or (II) above, they stand for the residues of the first and second analgesic moieties, respectively.

The foregoing and other needs are met by embodiments according to the present invention, which provide compounds of the following formula: 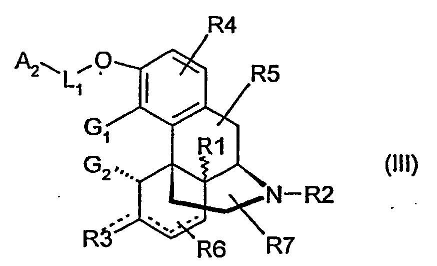 wherein A₂ is a second analgesic moiety;
L₁ is a direct bond or a linker;
Each ― is a single or a double bond;
R₁ is H, CH₃ or OH;
R₂ is H, C₁-C₆-alkyl, C₃-C₆-cycloalkyl-C₁-C₆-alkyl, C₁-C₆-alkenyl, C₁-C₆₋alkanoyl, C₃-C₆-cycloalkenyl-C₁-C₆-alkyl, C₃-C₆-cycloalkyl-C₁-C₆-alkanoyl, or C₃-C₆₋cycloalkenyl-C₁-C₆-alkanoyl;
R₃ is H, oxo (=O), hydroxyl (-OH), or C₁-C₁₂-alkanoyl, or -L₂-A₃;
wherein L₂ is a direct bond or a linker and A₃ is a residue of an analgesic compound, which may be the same as, or different from, A₁ and A₂;
R₄-R₇ are H, methyl, ethyl, F, Cl, Br, or l;
and G₁ and G₂ are each H or together represent the oxygen of a dihydrofurano ring or salts thereof.

A preferred class of compounds according to the present invention is represented by formula IV:

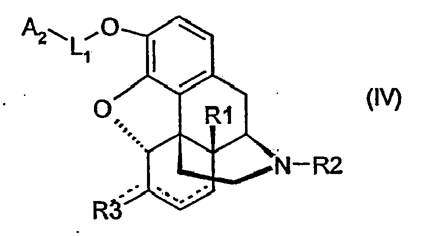

Wherein A₂, L₁, R₁ and R₃ are defined above, each ― is a single or double bond, and R₂ is methyl, cyclopropylmethyl, cyclobutylmethyl, or 3-propenyl.

An especially preferred class of compounds according to the present invention is represented by formula V: 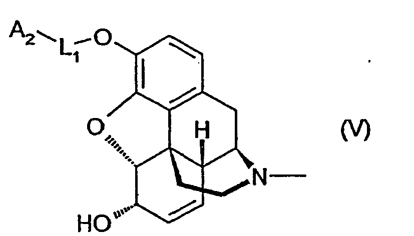 wherein A₂ and L₁ are defined above.

In some preferred embodiments according to the present invention, the A₂ moiety in formula V is a residue of morphine, naproxen, fluocinolone acetonide, aspirin, flurbiprofen, indomethacin, diclofenac and naproxen.

Another especially preferred class of compounds according to the present invention is represented by formula VI: 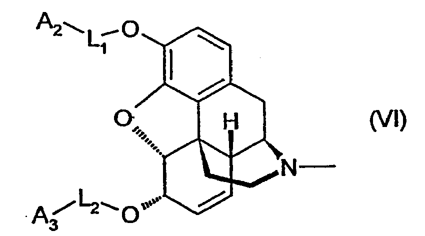 wherein A₂, A₃, L₁ and L₂ are defined above.

In some preferred embodiments according to the present invention, the A₂ and A₃ moieties are both naproxen residues. In other preferred embodiments, A₂ and A₃ are both diclofenac residues.

Compounds according to the present invention may be prepared in free form, or may be prepared as salts; such as mineral acid, carboxylic acid, or amine salts thereof. Compounds according to the present invention may be prepared as amorphous or crystalline forms, and may be in the form of anhydrates or hydrates. Compounds according to the present invention may be present as prodrugs, such as esters. In each of these cases, the critical feature is that a compound according to the present invention be stable under some conditions other than physiologic conditions, and be capable of decomposing under physiologic conditions to form first and second analgesic compounds, which analgesic compounds may be the same or different, as discussed above.

Compounds according to the present invention are synthesized in the manner illustrated in Schemes 1-6 below. In general, where the first and second analgesic moieties are to be directly linked, the first moiety is condensed with the second moiety under conditions suitable for forming a linkage that is labile under physiologic conditions. In some cases it is necessary to block some reactive groups on one, the other, or both of the analgesic moieties. Where the analgesic moieties are to be covalently linked via a linker, such as oxamethylene, succinic acid or diglycolic acid, it is advantageous to first condense the first analgesic moiety with the linker. In some cases it is advantageous to perform the reaction in a suitable solvent, such as acetonitrile, in the presence of suitable catalysts, such as carbodiimides including EDCI and DCC, or under conditions suitable to drive off water of condensation or other reaction products (e.g. reflux), or a combination of two or more thereof. After the first analgesic moiety is condensed with the linker, the combined first moiety and linker may then be condensed with the second analgesic moiety. Again, in some cases it is advantageous to perform the reaction in a suitable solvent, such as acetonitrile, in the presence of suitable catalysts, such as carbodiimides including EDCI and DCC, or under conditions suitable to drive off water of condensation or other reaction products (e.g. reflux), or a combination of two or more thereof. Where one or more active groups have been blocked, it may be advantageous to remove the blocking groups under selective conditions, however it may also be advantageous, where the hydrolysis product of the blocking group and the blocked group is physiologically benign, to leave the active groups blocked.

The person having skill in the art will recognize that, while diacids, dialcohols, amino acids, etc. are described above as being suitable linkers, other linkers are contemplated as being within the present invention. For instance, while the hydrolysis product of a compound according to the present invention may comprise a diacid, the actual reagent used to make the linkage may be, for example, a diacylhalide, such as succinyl chloride, or an anhydride, such as succinic anhydride or diglycolic anhydride. The person having skill in the art will recognize that other possible acid, alcohol, amino, sulfato, and sulfamoyl derivatives may be used as reagents to make the corresponding linkage.

Where the first and second analgesic moieties are to be directly linked via a covalent bond, essentially the same process is conducted, except that in this case there is no need for a step of adding a linker. The first analgesic moiety and second analgesic moieties are merely combined under conditions suitable for forming the covalent bond. In some cases it may be desirable to block certain active groups on one, the other, or both of the analgesic moieties. In some cases it may be desirable to use a suitable solvent, such as acetonitrile, a catalyst suitable to form the direct bond, such as carbodiimides including EDCI and DCC, or conditions designed to drive off water of condensation (e.g. reflux) or other reaction by-products.

The person having skill in the art will recognize that, while in most cases the first and second moieties may be directly linked in their original form, it is possible for the active groups to be derivatized to increase their reactivity. For instance, where the first moiety is an acid and the second moiety is an alcohol (i.e. has a free hydroxyl group), the first moiety may be derivatized to form the corresponding acid halide, such as an acid chloride or an acid bromide. The person having skill in the art will recognize that other possibilities exist for increasing yield, lowering production costs, improving purity, etc. of the compound according to the present invention by using conventionally derivatized starting materials to make compounds according to the present invention.

Exemplary reaction schemes according to the present invention are illustrated in schemes 1-6 below, and in the examples. The person of skill in the art will appreciate that these schemes may be generalized by using appropriate linkers and analgesic agents as starting materials.

A focus of the present invention is upon treatment of acute and chronic pain, especially pain associated with surgery, deep tissue trauma, tumors, lacerations and abrasions. As prevention of pain tends to be more effective than post hoc treatment, a focus of this present invention is upon forestalling or preventing pain. However, the compounds, compositions, articles and methods of the present invention may also be used to treat pain post hoc, for instance in post-trauma situations, or in post-operative situations where conventional pain treatment methods have been tried but have proven ineffective or intolerable.

An example of an instance where the compounds of the present invention would find utility, is in the context of post-operative pain management, In a typical instance, an individual has been subjected to local or general anesthesia, one or more incisions have been made in the individual's soft tissue, and the incision has been closed by one or more closures, for instance staples or sutures. Compounds according to the present invention may be directly implanted in a site in the vicinity of the surgical incision, in the vicinity of soft tissues that have been subjected to surgical or pre-surgical trauma, or both. In some embodiments according to the present invention, it may be desirable to combine a compound according to the present invention with one or more polymer vehicle. Such polymer vehicle may be any physiologically tolerated polymer, such as a bioerodible or a non-bioerodible polymer.

A polymer useful in a composition according to the present invention includes any biologically tolerated polymer that is permeable to a compound according to the present invention or that is permeable to the inventive compound and cleavage products thereof after the inventive compound has been cleaved, or that is bioerodible so that it releases the compound according to the present invention in a sustained-release manner. In some embodiments according to the present invention, the polymer has a permeability such that it is not the principal rate determining factor in the rate of release of the compound according to the present invention from the polymer. In some embodiments according to the present invention, the polymer is non-bioerodible. Examples of non-bioerodible polymers useful in the present invention include polyvinylalcohol and polyurethane. In other embodiments of the present invention, the polymer is bioerodible. Examples of bioerodible polymers useful in the present invention include polyanhydride, polylactic acid, polyglycolic acid, polyorthoester, polyalkylcyanoacrylate or derivatives and copolymers thereof. The skilled artisan will recognize that the choice of bioerodibility or non-bioerodibility of the polymer depends upon the final physical form of the system, as described in greater detail below. Other exemplary polymers include polysilicone and polymers derived from hyaluronic acid. The skilled artisan will understand that the polymer according to the present invention is prepared under conditions suitable to impart permeability such that it is not the principal rate determining factor in the release of the low solubility agent from the polymer.

Moreover, suitable polymers include naturally occurring (collagen, hyaluronic acid) or synthetic materials that are biologically compatible with bodily fluids and mammalian tissues, and essentially insoluble in bodily fluids with which the polymer will come in contact. In addition, the suitable polymers may also essentially prevent interaction between the low solubility agent dispersed/suspended in the polymer and proteinaceous components in the bodily fluid. The use of rapidly dissolving polymers or polymers' highly soluble in bodily fluid or which permit interaction between the low solubility agent and proteinaceous components are to be avoided since dissolution of the polymer or interaction with proteinaceous components would affect release of the drug.

Other suitable polymers include polypropylene, polyester, polyethylene vinyl acetate (PVA), polyethylene oxide (PEO), polypropylene oxide, polycarboxylic acids, polyalkylacrylates, cellulose ethers, polyalkyl-alkyacrylate copolymers, polyester-polyurethane block copolymers, polyether-polyurethane block copolymers, polydioxanone, poly-(β-hydroxybutyrate), polylactic acid (PLA), polycaprolactone, polyglycolic acid, polyethylene glycol (PEG) and PEO-PLA copolymers.

Further suitable polymers are set forth in US Patent No. 6,051,576, issued on April 18, 2000, to Ashton et al., which is expressly incorporated herein by reference.

The present invention includes an article comprising a compound according to the present invention, which article is suitable for implantation or injection into a site in the vicinity of a cause of pain. For instance, in the case of orthopedic surgery, an article according to the present invention may be a screw, having coated thereon, or implanted into a crevice or indentation therein, a compound according to the present invention, or a composition according to the present invention that comprises a suitable polymer and a compound according to the present invention. Other examples of articles according to the present invention are surgical staples, surgical anchors, stents, sutures, other types of surgical closures, bandages, absorbent pads, and prostheses. Additionally, an article according to the present invention may be an implantable capsule, or other device, adapted to contain a compound or composition according to the present invention for gradual release into the physiologic surroundings.

The present invention also provides methods for treating somatic pain. A method according to the present invention is useful for treating both nociceptive and neuropathic pain. In the case of nociceptive pain, a preferred mode of administration is for the practitioner to inject or implant a compound or composition into or near the locus that is the source of nociceptive pain stimulus. For instance, where the source of pain is a surgical incision, a compound or composition according to the present invention is advantageously implarited beneath the incision before the incision has been dosed. In another embodiment of the present invention, a compound or composition according to the present invention is injected subcutaneously in the vicinity of a surgical incision after the incision has been closed.

A method according to the present invention advantageously employs a compound or a composition according to the present invention, which may be delivered to an individual in need thereof in an art recognized manner, such as via intravenous, subcutaneous, intramuscular or other parenteral mode of injection, or by surgical implantation. Although intravenous injection is possible, the combined properties of the compounds and compositions according to the present invention make them particularly well-suited for subcutaneous or intramuscular implantation or injection into soft tissue in the vicinity of the origin of the pain stimulus. In an preferred embodiment according to the present invention, a compound according to the present invention is prepared in a solid form, such as a pellet that may be directly injected into the vicinity of the pain locus. In other embodiments according to the present invention, a compound according to the present invention is prepared in an anhydrous solution or suspension, for instance in vegetable oil, such as palm oil, and injected intramuscularly. In other embodiments according to the present invention, a compound according to the present invention is combined-with a polymer gel that may be bioerodible to form a composition according to the present invention, and the composition is injected intramuscularly or subcutaneously into tissue at or near the site that is the origin of pain.

The origin of the pain stimulus may be any sort of injury that is generally associated with pain, such as a surgically induced incision, a non-surgically induced insult, such as a contusion, an abrasion or a laceration, or a tumor.

Where the source of pain is a non-surgically induced tissue insult, such as an abrasion, a contusion or a laceration, a compound or composition according to the present invention is advantageously injected subcutaneously or intramuscularly into the vicinity of the insult.

Where the source of pain is a cancerous or non-cancerous tumor, a compound or composition according to the present invention is advantageously injected subcutaneously or intramuscularly into the vicinity of the tumor.

Where the source of pain is neuropathic, a compound or composition according to the present invention is advantageously injected into the vicinity of the malfunctioning neural fiber. The person skilled in the art will recognize that the malfunctioning neural fiber may first be detected using, for instance, instrumentation adapted to affect the TENS methodology of nerve fiber identification and location.

It is understood that, while the present invention is primarily contemplated for humans, it is also contemplated for use in veterinary medicine

The following examples illustrate the synthesis of compounds according to the present invention, and effects of the present invention.

### EXAMPLES

### Example 1

### Inventive compound (1) {morphine with 1 equivalent of naproxen} (Scheme 1)

To a stirred suspension of morphine (50 mg, 0.175 mmol) in acetonitrile (3 ml) under argon was added naproxen (44 mg, 0.192 mmol) followed by EDCI (37 mg, 0.192 mmol) and a catalytic amount of DMAP. The resulting cloudy mixture was stirred at room temperature overnight and the solvent was removed in vacuo. The residue was dissolved in ethyl acetate and subsequently washed with water, sodium bicarbonate solution, water, brine and dried over sodium sulfate. The crude produce after solvent evaporation was purified by column chromatography on silica gel to yield 45 mg of the inventive compound (1). ¹H-NMR (CDCl₃), 1.65 (d, 3H), 2.40 (s, 3H), 3.88 (s, 3H), 4.10 (m, 2H), 4.85 (d, 1H), 5.26 (m, 1 H), 5.74 (m, 1 H), 6.55 (d, 1H), 6.67 (d, 1 H), 7.12 (m, 2H), 7.48 (dd, 1H), 7.74 (m, 3H).

### Example 2

### Diester inventive compound (2) {morphine with naproxen} (Scheme 1)

To a stirred suspension of morphine (89 mg) in acetonitrile (5 ml) under argon was added naproxen (180 mg, 2.5 eq.) followed by EDCI (150 mg, 2.5 eq.) and a catalytic amount of DMAP. The reaction was performed and product isolated as described in Example 1 to afford diester inventive compound (2) (173 mg). ¹H-NMR (CDCl₃), 1.58 (d, 3H), 1.60 (d, 3H), 2.41 (s, 3H), 3.86 (s, 3H), 3.88 (s, 3H), 5.12 (m, 2H), 5.36 (m, 1H), 5.54 (m, 1 H), 6.51 (d, 1 H), 6.60 (d, 1 H), 7.07 (m, 4H), 7.44 (m, 2H), 7.66 (m, 6H).

### Example 3

### Inventive compound (4) {Diester of morphine with succinic acid} (Scheme 2)

Morphine (50 mg, 0.175 mmol) was dissolved in 1.5 ml of anhydrous pyridine at room temperature and DMAP (3 mg) was added to the solution. The reaction mixture was left at room temperature overnight and evaporated to dryness. The crude residue containing the monoester (3) was coevaporated with toluene to remove traces of pyridine and then dissolved in 3.5 ml of anhydrous acetonitrile. Morphine (50 mg) was added followed by EDCI (34 mg, 0.175 mmol) and DMAP (2 mg). The reaction mixture was stirred at room temperature overnight and evaporated to dryness. The residue was dissolved in ethyl acetate, washed with water, sodium bicarbonate solution, water and brine. Solvent evaporation left the crude product, which was purified by column chromatography to afford 25 mg of the inventive compound (4) as colorless foam. ¹H-NMR (CDCl₃), 2.44 (s, 6H), 3.00 (s, 4H), 3.35 (m, 2H), 4.16 (m, 2H), 4.90 (d, 2H), 5.26 (m, 2H), 5.73 (m, 2H), 6,58 (d, 2H), 6,76 (d, 2H).

### Example 4

### Inventive compound (5) {morphine with fluocinolone acetonide} (Scheme 3)

To a solution of morphine (40 mg, 0.14 mmol) in anhydrous pyridine (1.5 ml) under argon was added fluocinolone acetonide chloroformate (86 mg, 0.17 mmol) at room temperature. The resulting mixture was stirred overnight and the solvent was evaporated in vacuo. The residue was dissolved in ethyl acetate and worked up as described previously. Chromatography on silica gel afforded 43 mg of the inventive compound (5). ¹H-NMR (CDCl₃), 0.94 (s, 3H), 1.22 (s, 3H), 1.44 (s, 3H), 1.51 (s, 3H), 2.45 (s, 3H), 3.06 (d, 1 H), 3.37 (m, 1 H), 3.94 (m, 1 H), 4.21 (m, 1 H), 4.41 (m, 2H), 4,78 (d, 1 H), 4,97 (m, 2H), 5.05 (d, 1 H), 5.26 (m, 2H), 5.60 (d, 1 H), 6.36 (dd, 1H), 6.42 (s, 1 H), 6.64 (d, 1 H), 6.90 (d, 1H), 7.19 (d, 1 H).

### Example 5

### inventive compound (6) {fluocinolone acetonide with morphine linked via diglycolic acid moiety} (Scheme 3)

Following the procedure outlined in Example 1, the inventive compound (6) was prepared from morphine (59 mg, 0.207 mmol), fluocinolone acetonide hemidiglycolate (83 mg, 0.207 mmol), EDCI (40 mg, 0.207 mmol) and DMAP in acetonitrile (3 ml). Chromatographic purification yielded 55 mg of the pure product. ¹H-NMR (CDCl₃), 0.94 (s, 3H), 1.21 (s,-3H); 1.42 (s, 3H), 1.52 (s, 3H), 2.44 (s,3H), 3.40 (m,2H), 4.18 (m, 1 H), 4.44 (m, 4H), 5.31 (m, 1H), 5.69 (m, 1H), 6.37 (dd, 1H), 6.43 (s, 1 H), 6.63 (d, 1H), 6.78 (d, 1H), 7.15 (dd, 1H).

### Example 6

### Inventive compound (7) {morphine with ethacrynic acid} (Scheme 4)

Following the procedure of Example 1, the inventive compound (7) was prepared from morphine (80 mg, 0.280 mmol), ethacrynic acid (93 mg, 0.308 mmol), EDCI (59, mg, 0.308 mmol), a catalytic amount of DMAP in acetonitrile (4 ml). 71 mg of the product was obtained after column chromatography. ¹H-NMR (CDCl₃), 1.5 (t, 3H), 2.44 (s, 3H), 2.45 (q, 2H), 3.36 (m, 1H), 4.16 (broad s, 1H), 4.92 (d, 1H), 5.01 (s, 2H), 5.27 (m, 1H), 5.64 (s, 1H), 5.71 (m, 1 H), 6.62 (d, 1 H), 6.75 (d, 1H), 6.89 (d, 1 H), 7.16 (dd, 1H).

### Example 7

### Inventive compound (8) {morphine with aspirin} (Scheme 4)

Following the procedure of Example 1, the inventive compound (8) was prepared from morphine (80 mg, 0.280 mmol), acetylsalicylic acid (8 ml). The ester (8) was obtained as colorless foam (44 mg). ¹H-NMR (CDCl₃), 2.34 (s, 3H), 2.44 (s, 3H), 3.08 (d, 1H), 3.39 (m, 1H), 4.18 (m, 1H), 4.95 (d, 1H), 5.30 (m, 1 H), 5.79 (m, 1 H), 6.64 (d, 1 H), 6.83 (d, 1 H), 7.16 (dd, 1H), 7.37 (m, 1H), 7.63 (m, 1H), 8.24 (dd, 1H).

### Example 8

### Inventive compound (9) {flurbiprofen with morphine} (Scheme 4)

Following the procedure of Example 1, the inventive compound (9) was prepared from morphine (80 mg, 0.280) flurbiprofen (75 mg, 0.308 mmol), EDCI (59 mg, 0.308 mmol) and a catalytic amount of DMAP in acetonitrile (4 ml). The reaction was performed at 0-5°C overnight to give 55 mg overnight to give 55 mg of the ester (10) after chromatographic purification. ¹H-NMR (CDCl₃), 2.43 (s, 3H), 2.68 (m, 1H), 3.04 (d, 1H). 3.37 (m, 1H), 4.05 (d, 2H), 4.12 (m, 1H), 4.87 (dd, 1H), 5.24 (m, 1 H), 5.70 (m, 1 H), 6.57 (d, 2H), 6.71 (s, 1H), 6.74 (d, 1H), 6.97 (m, 2H), 7.15 (_, 1H), 7.32 (d, 2H).

### Example 10

### Inventive compound (11) {morphine with indomethacin} (Scheme 5)

Following the procedure of Example 1, the inventive compound (11) was prepared from morphine (80 mg, 0.280 mmol), indomethacin (110 mg (0.308 mmol), EDCI (59 mg, 0.308 mmol) and a catalytic amount of DMAP in acetonitrile (4 ml). Column chromatography of the crude mixture afforded 169 mg of the pure inventive compound. ¹H-NMR (CDCl₃), 2.42 (s, 3H), 2.43 (s, 3H), 2.60 (m. 1 H), 2.64 (m, 1 H), 3.02 (d, 1H), 3.35 (m, 1H), 3.85 (s, 3H), 3.90 (s, 2H), 4.14 (d, 1H), 7.03 (d, 1H). 7.47 . (d, 2H), 7.67 (d, 2H).

### Examples 11 and 12

### Inventive compounds (12) and (13) {naproxen with morphine} (Scheme 6)

Inventive compounds (12) and (13) with naproxen linked to morphine via oxa acids linkages were prepared following the procedure of Example 1 using the corresponding ester prodrugs of naproxen in the presence of EDCI and DMAP. inventive compound (12) was isolated with 45% of yield as colorless foam and inventive compound (13) was obtained with 60% of yield. (12) ¹H-NMR (CDCl₃), 1.58 (d, 3H, 2.44 (s,3H), 3.59 (t, 2H), 3.64 (t, 3H), 3.89 (2, 3H), 4.25 (2, 2H), 4.94 (d, 1H), 5.26 (d, 1 H), 5.72 (d, 1H), 6.59 (d, 1H), 6.80 (d, 1H), 7.10 (m, 2H), 7.42 (dd, 1H), 7.69 (m, 3H). (13) ¹H-NMR (CDCl₃), 1.56 (d, 3H), 2.43 (s, 3H), 3.87 (s, 3H), 4.30 (s, 2H), 4.89 (d, 1H), 5.27 (m, 1H), 5.71 (d, 1H), 6.58 (d, 1 H), 6.74 (d, 1H), 7.11 (m, 2H), 7.40 (m, 1 H), 7.68 (m, 3H).

### Example 13

### Inventive compound (14) {morphine with naproxen linked via salicylic acid} (Scheme 6)

Following the procedure outlined in Example 1, the ester (14) was prepared from morphine (58 mg, 0.202 mmol), naproxen ester of salicylic acid (78 mg, 0.222 mmol), EDCI (0.222 mmol), and DMAP in acetonitrile (2 ml). Typical work-up followed by preparative thin layer chromatography afforded 38 mg of the Inventive compound (14). ¹H-NMR (CDCl₃), 1.67 (d, 3H), 2.45 (s, 3H), 3.91 (s, 3H), (s, 3H), 4.16 (m, 2H), 4.93 (d, 1 H), 5.30 (m, 1 H), 5.78 (m, 1H), 6.58 (d, 1 H), 6.75 (d, 1H), 6.93 (m, 1H), 7.10 (m, 2H), 7.32 (m, 1H), 7.52 (m, 2H), 7.70 (m, 3H), 8.18 (dd, 1H).

### Example 14

### Inventive compound (16) {morphine with naproxen linked via formaldehyde linkage} (Scheme 7)

Morphine (100 mg, 0.35 mmol) was suspended in 1.5 ml of anhydrous acetonitrile at room temperature under argon. Triethylamine (54 µl) was then added and the mixture was cooled in an ice bath. Chloromethyl chloroformate (50 mg, 0.385 mmol) was then slowly added via syringe under argon. The reaction mixture was stirred at 0-5°C for 1 hr. and then at room temperature overnight The product was poured into excess cold water and the product was extracted with ethyl acetate. The organic solution was washed with water, brine, dried over Na₂SO₄ and evaporated to yield the crude chloromethyl carbonate of morphine (15). The ester (15) was then dissolved in HMPA (1.5 ml) and sodium salt of naproxen (57 mg, 0.224 mmol) was added under argon. The cloudy mixture was then stirred at room temperature overnight Addition of icy water precipitated the crude product, which was separated by filtration and dried in a vacuum desiccator. Purification by preparative thin layer chromatography afforded 19 mg of inventive compound (16). ¹H-NMR (CDCl₃), 1.62 (d, 3H), 2.44 (s, 3H), 3.04 (d, 1H), 3.36 (m, 1 H), 3.91 (s, 3H), 4.16 (m, 1H), 4.80 (d, 1 H), 4.92 (d, 1 H), 5.26 (m, 1 H), 5.72 (m, 1H), 5.83 (q, 2H), 6.60 (m, 2H), 7.11 (m, 2H), 7.40 (m, 1 H), 7.69 (m, 3H).

### Example 15

### Codrug (10) of morphine with diclofenac (Scheme 5)

Following the procedure of Example 1, the codrug (10) was prepared from morphine (80 mg, 0.280 mmole), diclofenac (91 mg, 0.308 mmole), EDCI (59 mg, 0.308 mmole) and catalytical amount of DMAP in acetonitrile (4 mL). The reaction was performed at 0-5°C overnight to give 55 mg of the ester (10) after chromatographic purification. ¹H-NMR (CDCl₃), 2.43 (s, 3H), 2.68 (m, 1H), 3.04 (d, 1H), 3.37 (m, 1H), 4.05 (d, 2H), 4.12 (m, 1H), 4.87 (dd, 1H), 5.24 (m, 1H), 5.70 (m, 1H), 6.57 (d, 2H), 6.71 (s, 1 H), 6.74 (d, 1H), 6.97 (m, 2H), 7.15 (, 1H), 7.32 (d, 2H).

### Example 16

### Stability of inventive compound (9) {flurbiprofen with morphine}

A stock solution (1 mg/ml) of inventive compound (9) was prepared in acetonitrile. This stock solution was diluted 10 fold in either 0.1 M phosphate buffer, pH 7.4, or human plasma, and the working solution was placed in a water bath at 37°C. Samples had been taken every hour for the plasma and every couple of hours for the buffer. After proper sample preparation, the inventive compound and parent drugs were analyzed with a HPLC system that includes Hitachi software, a pump, an autosample, and a UV detector. For inventive compound (9) a Supelcosil LC-ABZ column (5 cm x 4.6 mm) was used and the mobile phase was a mixture of 63 % and 37 % of 0.01 M phosphate buffer, pH 7.4, and the flow rate was 1.5 ml/min, and the detector was set at 246 nm.

The half-life of inventive compound (9) in human plasma is 7.4 hours, and the half-life in 0.1 M Phosphate buffer (pH 7.4) is 43 hours.

Similar procedures have been used for determining the stability of other morphine inventive compounds. Modifications were applied in each individual inventive compound. The following table summarizes the stability results of the morphine Inventive compounds.

| Compound | Half-Life in Human Plasma | Half-Life in Phosphate Buffer, pH 7.4 |
|---|---|---|
| Inventive compound (1) | 70 ± 10 min. | 50 ± 5 hr. |
| Inventive compound (5) | 69 ± 1 min. | |
| Inventive compound (6) | 7.5 ±0.5 min. | 68 ± 3 hr. |
| Inventive compound (8) | 90 ± 10 min. | 60 ± 5 hr. |
| Inventive compound (9) | 7.4 ±0.1 hr. | 43 ± 5 hr. |
| Inventive compound (10) | 21 ± 1 min. | 2.5 ± 0.5 hr. |
| Inventive compound (11) | 102 ± 5 min. | |
| Inventive compound (14) | 26 ± 4 min. | 35 ± 5 hr. |
| Inventive compound (16) | 3.5 ± 0.6 min. | 7.6 ± 0.5 hr. |

### Synthetic Schemes

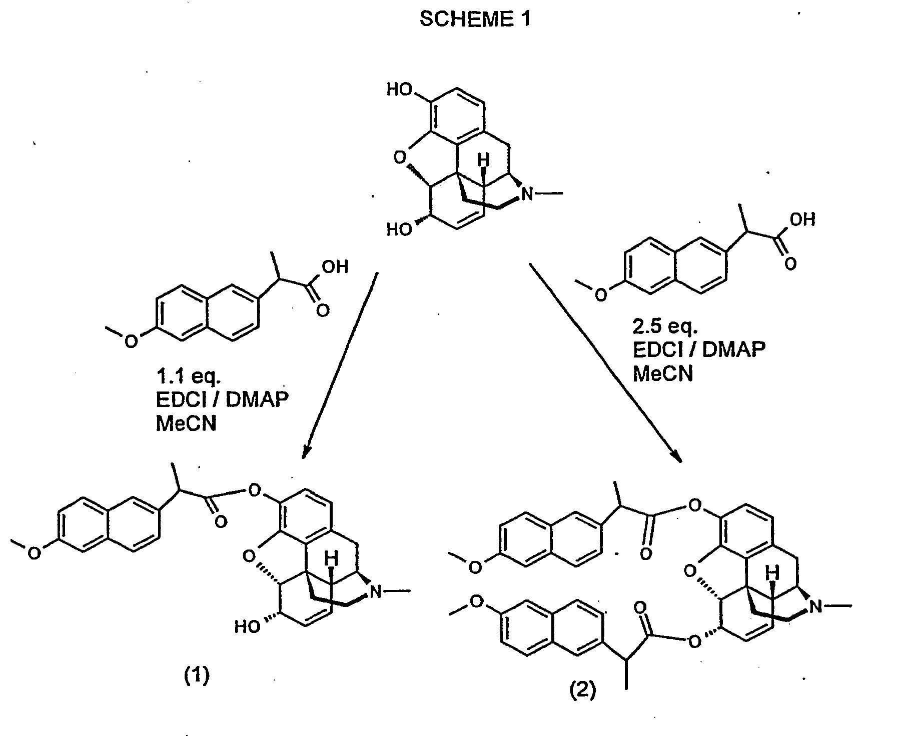

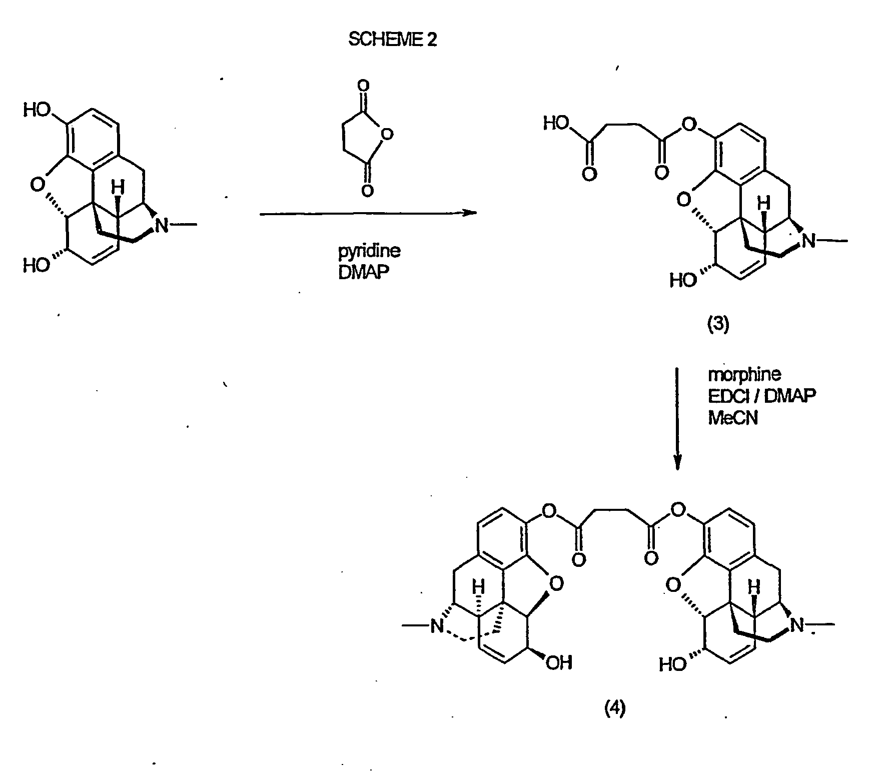

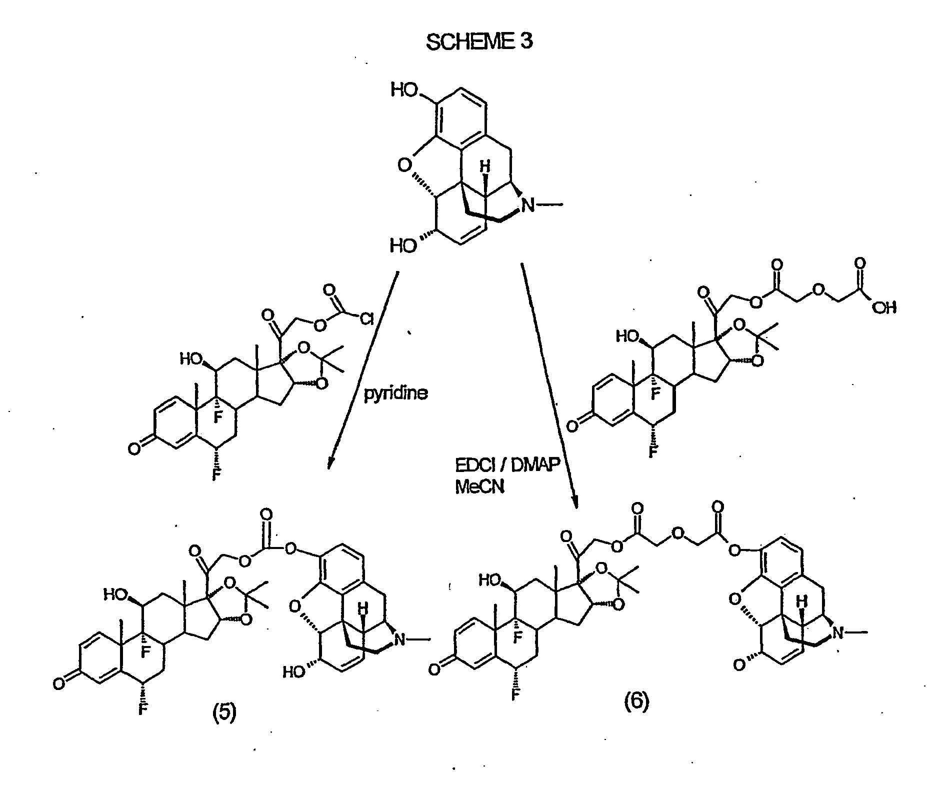

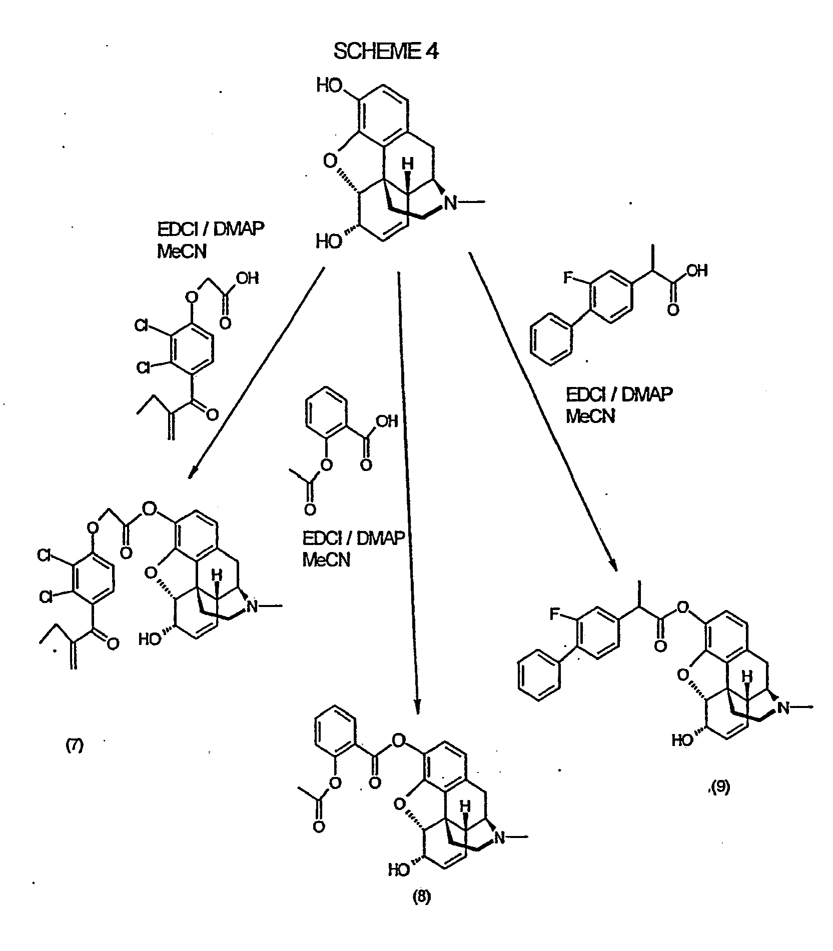

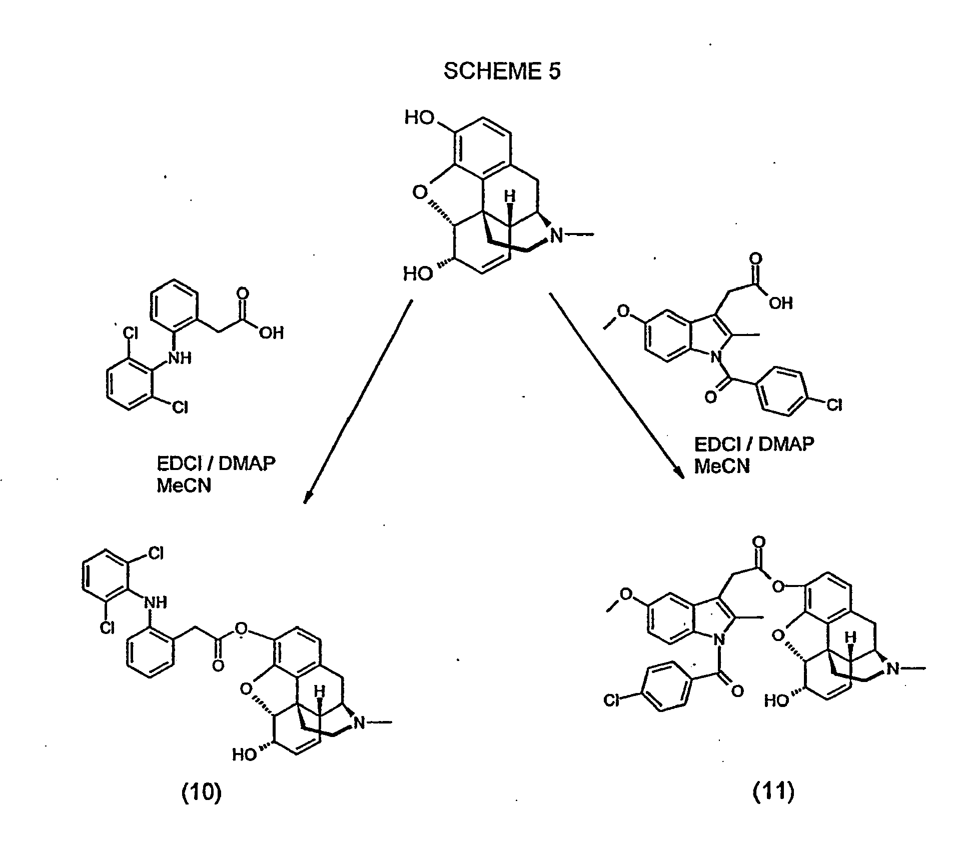

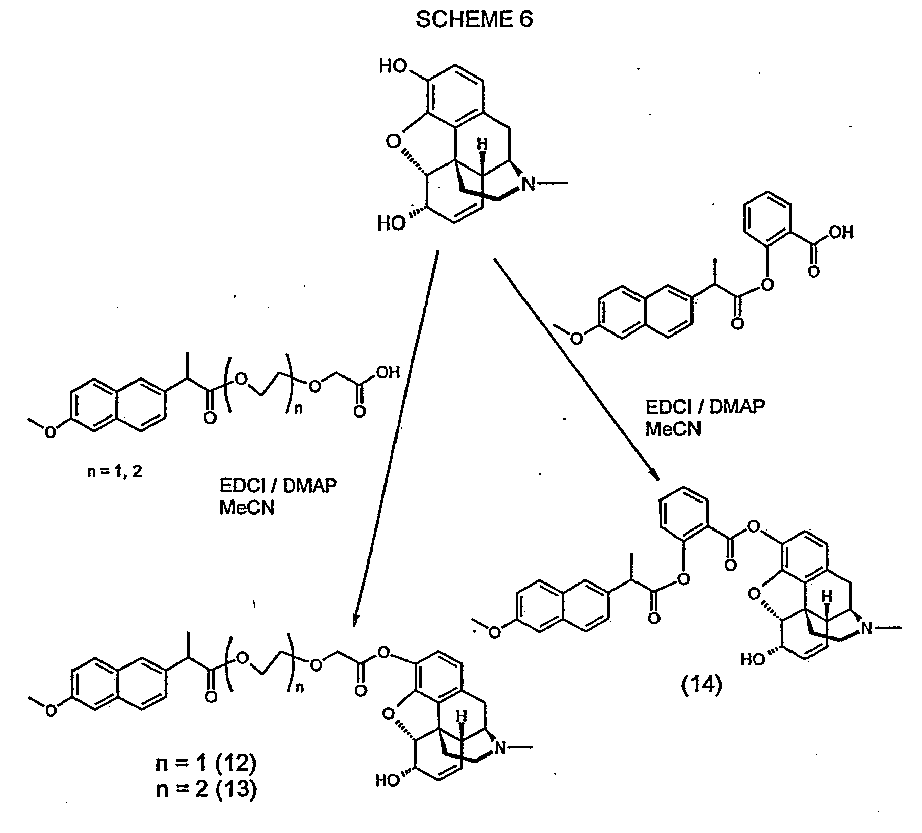

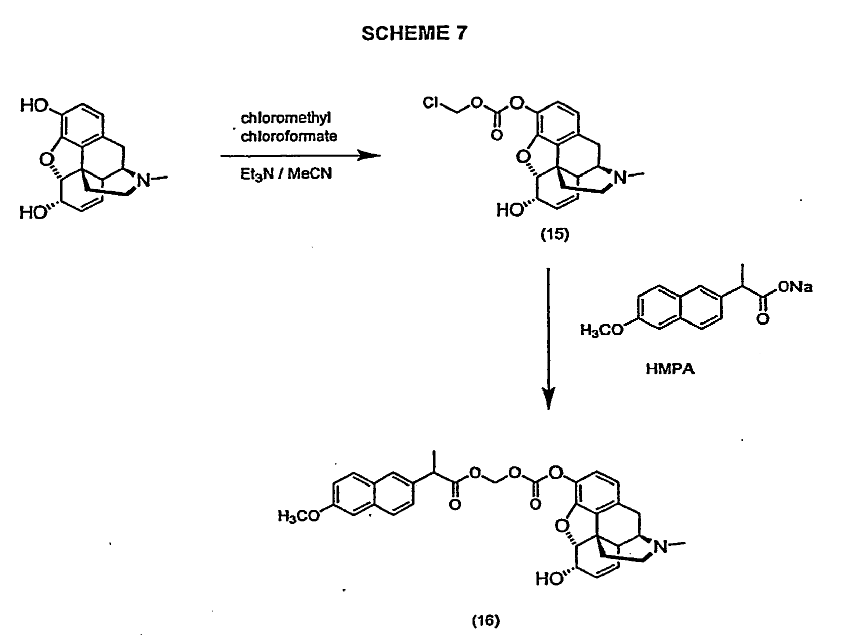

### Analgesic Compounds Useful in the Present Invention

### Opioid Analgesic

### Morphine-Like Analgesics

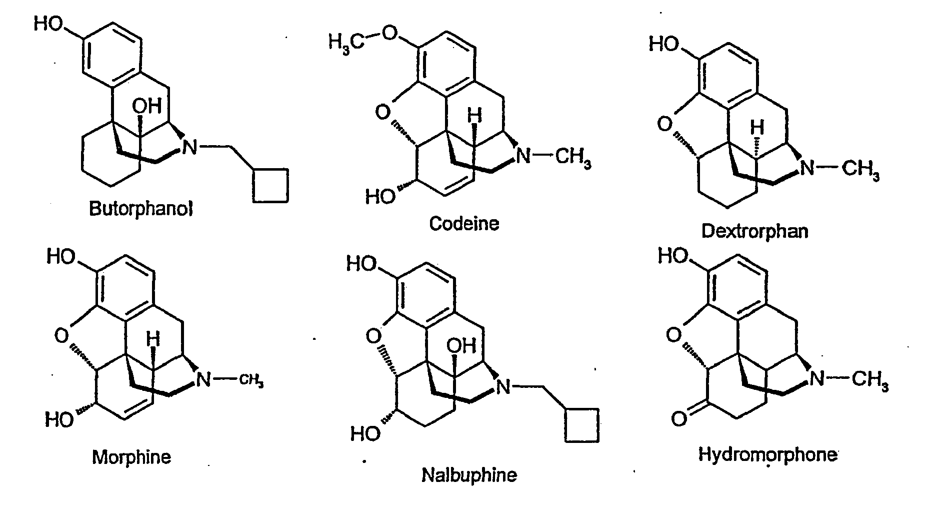

### More Opioid Analgesics

### More Morphine-Like Analgesics

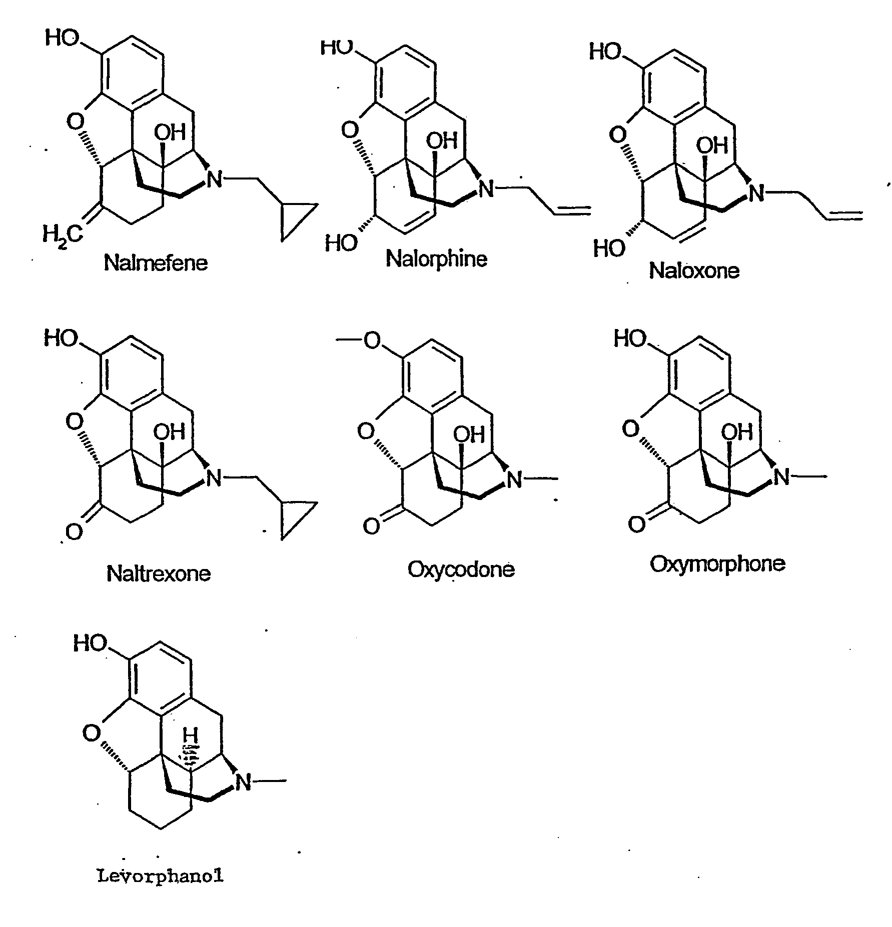

### NSAIDs (Non-Steroidal Anti-inflammatory Agents)

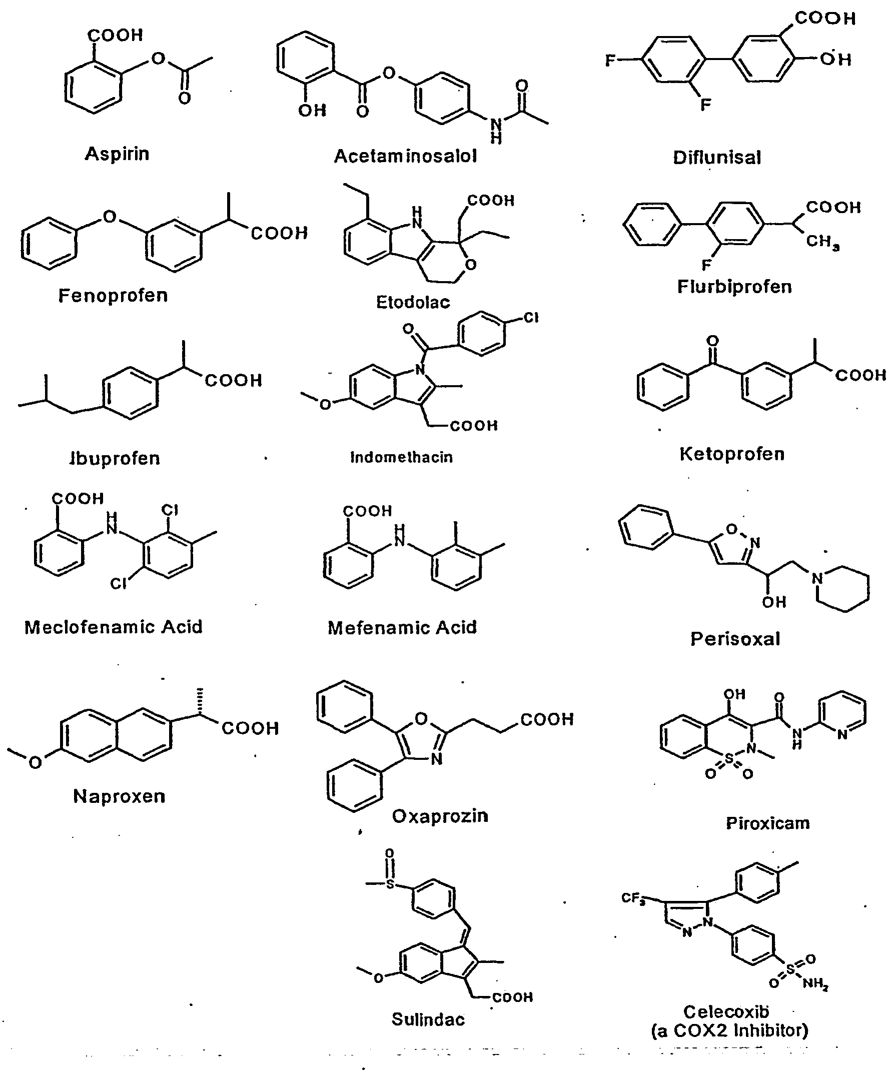

### Narcotics

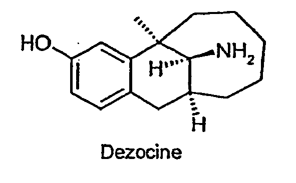

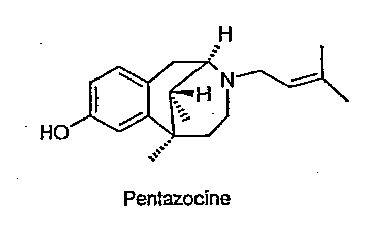

### Other Analgesics

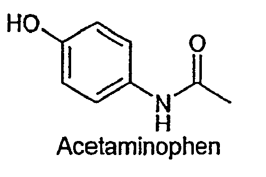

### Steroidal Anti-inflammatories

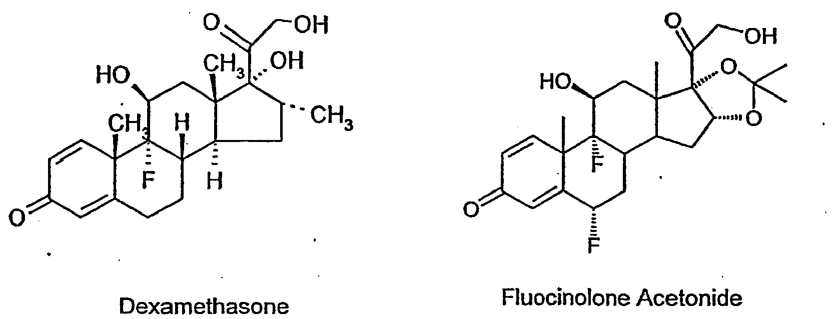

The above description is meant to be illustrative only of the present invention, and not limiting thereof. Other variations of method, composition, and manufacture will become apparent to those skilled in the art upon consideration of the foregoing specification and the appended claims.

## Claims

1. A sustained release analgesic system comprising a prodrug having a general formula of A-L-B in which: A represents an non-steroidal anti-inflammatory drug (NSAID) moiety or an opioid drug moiety having a therapeutically active form for producing an analgesic response in a patient; L represents a covalent linker linking A and B to form a prodrug, said linker being cleaved under physiological conditions to generate said therapeutically active form of A; and B represents a moiety which, when linked to A, results in the prodrug having a lower solubility than the therapeutically active form of A alone.

2. A system according to claim 1, wherein the linkage L is hydrolyzed in bodily fluid.

3. A system according to claim 1, wherein the linkage L is enzymatically cleaved.

4. A system according to claim 1, wherein L includes one or more hydrolyzable groups selected from an ester, an amide, a carbamate, a carbonate, a cyclic ketal, a thioester, a thioamide, a thiocarbamate, a thiocarbonate, a xanthate and a phosphate ester.

5. A system according to claim 1, wherein the system is a composition and is sterile and pyrogen free.

6. A system according to claim 1, wherein the therapeutically active form of A is at least 5 times more soluble in water relative to said prodrug.

7. A system according to claim 1, wherein the therapeutically active form of A has a logP value at least 0.5 logP unit less than the logP value of the prodrug.

8. A system according to claim 1, wherein the prodrug, in its linked form, has an ED50 for producing analgesia at least 10 times greater than the ED50 of the therapeutically active form of A.

9. A system according to claim 1, wherein the prodrug per se is inert with respect to inducing analgesia.

10. A system according to claim 1, wherein B is a hydrophobic aliphatic moiety.

11. A system according to claim 1, wherein B, after cleavage from the prodrug, can be a biologically inert moiety.

12. The system of claim 1, wherein A is an NSAID moiety represented by the general formula: 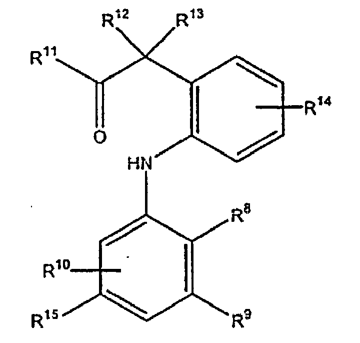 wherein
R⁸ is a lower alkyl, a lower alkoxy, a fluoro, a chloro;
R⁹ and R¹⁰ are each, independently for each occurrence, a hydrogen, a lower alkyl, a fluoro, a chloro, or a trifluoromethyl;
R¹¹ is a hydrogen, a lower alkyl or benzyl;
R¹² is a hydrogen or a lower alkyl;
R¹³ is a hydrogen, a lower alkyl or when R¹² is hydrogen, benzyl;
R¹⁴ is a hydrogen, a lower alkyl, a lower alkoxy, a fluoro, a chloro, or a bromo;
R¹⁵ is hydrogen or trifluoromethyl when R⁸ is hydrogen or chloro and R⁹ is hydrogen or trifluoromethyl.

13. A sustained release analgesic system comprising a prodrug having a general formula of A::B in which A represents an NSAID or opioid drug moiety having a therapeutically active form for producing an analgesic response in a patient; "::" represents an ionic bond between A and B that dissociates under physiological conditions to generate said therapeutically active form of A; and B represents a moiety which, when ionically bonded to A, results in the prodrug having a lower solubility than the therapeutically active form of A.
